(19) Europäisches Patentamt
European Patent Office
Office européen des brevets

(11) Numéro de publication: **0 172 376**
**A1**

(12) **DEMANDE DE BREVET EUROPEEN**

(21) Numéro de dépôt: **85108418.6**

(22) Date de dépôt: **06.07.85**

(51) Int. Cl.[4]: **C 07 D 303/06**
A 61 K 7/46, C 07 D 301/14
C 07 B 37/10
//C07C13/553

(30) Priorité: **26.07.84 CH 3626/84**

(43) Date de publication de la demande:
**26.02.86 Bulletin 86/9**

(84) Etats contractants désignés:
**CH DE FR GB LI NL**

(71) Demandeur: **FIRMENICH SA**
**1, route des Jeunes**
**CH-1211 Genève 8(CH)**

(72) Inventeur: **Mazenod, François, Dr.**
**16, Cité Vieusseux**
**CH-1203 Geneve(CH)**

(72) Inventeur: **Giersch, Wolfgang Klaus**
**323, rue de Bernex**
**CH-1233 Bernex(CH)**

(74) Mandataire: **Salvadori, Giuseppe, Dr.**
**c/o Firmenich S.A. Case Postale 239**
**CH-1211 Genève 8(CH)**

(54) **Epoxyde tricyclique et son utilisation à titre d'ingrédient parfumant.**

(57) L'époxyde tricyclique obéit à la formule

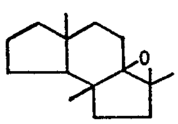

(I),

lequel composé possède des caractères odorants de type boisé-ambré. Il sert à la préparation de parfums et produits parfumés.

Procédé pour sa préparation à partir de triméthyl-cyclododécatriène.

Firmenich

# Epoxyde tricyclique et son utilisation à titre d'ingrédient parfumant

La présente invention a trait au domaine de la parfumerie. Elle concerne en particulier un composé oxygéné tricyclique de formule

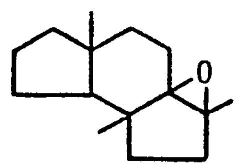

(I)

défini comme le 3,3a-époxy-perhydro-3,5a,8b-triméthyl-cyclopent[e]indène.

Nous avons en effet découvert que ledit composé possède des propriétés odorantes fort avantageuses et que de ce fait il peut trouver un emploi étendu dans différentes applications de parfumerie. Il sert tout spécialement à développer des notes boisées de type bois de cèdre, ainsi que des notes ambrées. Son caractère évoque, de par sa note chaude, l'odeur très naturelle de bois de pin. Doté d'une bonne puissance et de ténacité, il trouve une utilisation variée tant en parfumerie alcoolique que dans des applications techniques tel le parfumage de savons, détergents, revitalisants textiles, assainisseurs d'air ambiant, shampoings ou produits d'entretien. Il convient également au parfumage des produits cosmétiques tels les laits de beauté, les crèmes, les produits moussants pour bain ou les produits capillaires.

A titre de détergents, on entend indifféremment les détergents anioniques, cationiques, non ioniques ou zwitterioniques, tant destinés au lavage du linge qu'à celui de la vaisselle.

Les proportions dans lesquelles le composé oxygéné de l'invention peut produire les effets désirés varient dans une gamme de valeurs assez étendue. C'est ainsi que des concentrations de l'ordre de 0,1-0,5% en poids, par rapport au poids de l'article parfumé, peuvent parfaitement convenir au parfumage d'articles tels les savons ou les détergents. Des concentrations supérieures, par exemple de

5-10, voire 20% en poids peuvent être employées lors de la manufacture de compositions parfumantes concentrées. Comme à l'accoutumée, de telles valeurs de concentrations ne doivent pas être interprétées de façon limitative, l'homme de l'art sait en effet par expérience que des variations peuvent être apportées en fonction des autres constituants dans une composition parfumante donnée ou en fonction de la nature des produits que l'on désire parfumer. Le composé oxygéné de l'invention peut être employé soit à l'état isolé, soit en mélange avec d'autres ingrédients parfumants, de solvants, diluants ou supports. A titre d'ingrédients parfumants courants, il convient de citer les composés naturels, d'origine animale ou végétale, ou synthétiques dont de nombreux exemples ont été cités dans l'art antérieur (voir par exemple la demande de brevet européen no. 0096243).

Le composé oxygéné de l'invention est une entité chimique nouvelle. Il peut être obtenu conformément à un procédé original à partir de triméthyl-cyclododécatriène par cyclisation en milieu acide et époxydation. A titre d'oléfine de départ, on utilise un mélange commercial constitué pour l'essentiel par le 1,5,10-triméthyl-cyclododéca-1c,5t,9t-triène, lequel composé est accompagné par des quantités inférieures de 1,5,9-triméthyl-cyclododéca-1c,5t,9t-triène et 1,5,9-triméthyl-cyclododéca-1t,5t,9t-triène ainsi que d'autres oligomères provenant de la trimérisation d'isoprène.

Le procédé dont il est fait état plus haut peut être illustré par le schéma réactionnel suivant :

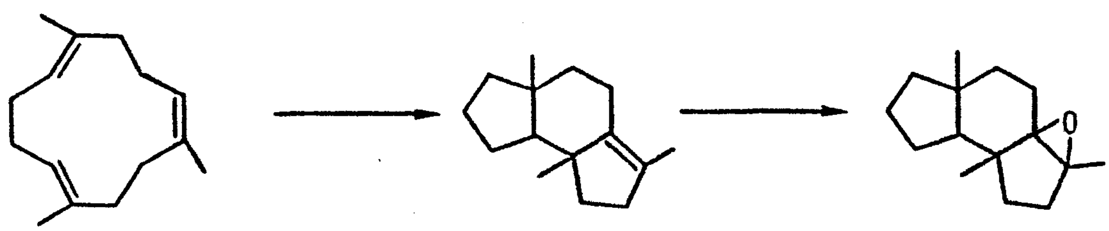

Quoique l'analyse spectrale de l'oléfine tricyclique intermédiaire suggère la structure indiquée, d'autres structures peuvent être formulées sur la base de considérations mécanistiques. Comme indiqué plus haut, le produit de départ utilisé est constitué par un mélange de différentes oléfines isomériques ; dès lors il n'est pas surprenant de constater que l'époxyde obtenu est accompagné par des quantités variables de composés isomériques. Bien entendu, le produit peut être isolé à l'état pur par l'emploi des techniques usuelles de séparation et purification, telle par exemple la chromatographie préparative en phase gazeuse. Une telle séparation cependant s'est révélée comme n'étant pas indispensable, les qualités olfactives du mélange convenant parfaitement pour toute utilisation pratique. C'est ainsi que le mélange obtenu par la cyclisation acide du triméthyl-cyclododécatriène de départ est directement époxydé à l'aide des méthodes usuelles par l'emploi d'acides variés, tels par exemple l'acide performique, peracétique, perbenzoïque, m-chloro-perbenzoïque ou monoperphtalique [voir à cet effet : Modern Synthetic Reactions, H. O. House, W. A. Benjamin, Inc. (1972)].

La cyclisation de l'oléfine de départ est effectuée au moyen d'un agent cyclisant acide. A cet effet, on peut utiliser des acides carboxyliques ou minéraux ou un mélange des deux. C'est ainsi que des mélanges d'acide acétique et d'acide sulfurique, par exemple dans des proportions respectives de 2:1 à 20:1, peuvent être employés. La réaction de cyclisation s'effectue à une température légèrement supérieure à la température ambiante et de préférence sous une atmosphère de gaz inerte. Comme il a été indiqué plus haut, le mélange de cyclisation obtenu est utilisé sans purification ultérieure pour l'étape suivante d'époxydation.

L'invention est illustrée de manière plus détaillée par les exemples suivants dans lesquels les températures sont indiquées en degrés centigrades et les abréviations ont le sens commun dans l'art.

## Exemple 1

Préparation de 3,3a-époxy-perhydro-3,5a,8b-triméthyl-cyclopent[e]indène

a. Un mélange de triméthyl-cyclododécatriène constitué par 88% de 1,5,10-triméthyl-cyclododéca-1c,5t,9t-triène, 5% de 1,5,9-triméthyl-cyclododéca-1c,5t,9t-triène et 2% de 1,5,9-triméthyl-cyclododéca-1t,5t,9t-triène accompagnés de 5% d'autres oligomères (55,5 g ; 0,27 Mole) a été placé dans un réacteur de 250 ml muni d'un réfrigérant et d'un agitateur magnétique. On a ensuite ajouté 16,65 g (0,27 M) d'acide acétique (96%) et 2,8 g (28,6 mM) d'acide sulfurique concentré. Le mélange a été chauffé sous argon à $40^o$ pendant 5 h, puis il a été dilué dans du pentane, neutralisé avec NaOH à 30%, lavé à l'eau, séché sur $MgSO_4$ et distillé sur une colonne Vigreux. On a ainsi obtenu 44,9 g d'un mélange complexe (rend. 80,9%). Une séparation au moyen de chromatographie en phase gazeuse (colonne : CARBOWAX 15%, 300 cm, $180^o$) a permis d'obtenir un produit dont les caractères spectraux étaient les suivants :

RMN : $^1H$ (360 MHz) : 0,86 (6H) ; 1,53 (3H, 1s) ; 2,26 (1H, t) ; 1,3-1,45 (6H, m) ; 1,57-1,90 (8H, m) δ ppm ;

$^{13}C$ (90 MHz) : 16,96(q) ; 18,9(q) ; 20,7(q) ; 22,7(t) ; 26,2(t) ; 28,8(t) ; 31,1(d) ; 32,6(t) ; 32,9(t) ; 37,3(t) ; 39,2(t) ; 53,3(s) ; 56,9(s) ; 121,6(s) ; 139,7(s) δ ppm ;

SM : $M^+$ = 204 ; m/e : 189(45), 162(100), 161(65), 147(88), 133, 119, 105, 91, 79, 67, 55, 41.

Lorsqu'on effectue la cyclisation sur des quantités supérieures d'oléfine de départ (110 g), on obtient le produit désiré avec des rendements analogues, cependant la réaction s'effectue avec dégagement de chaleur et l'on opère par conséquent en refroidissant le réacteur afin de maintenir la température à une valeur inférieure à 40-50°.

b. Le mélange obtenu (4,1 g ; 20 mM) a été dilué dans 200 ml de $CH_2Cl_2$, puis à froid on a ajouté par portions 4,84 g (24 mM) d'acide m-chloro-perbenzoïque (85%). Après l'introduction, le mélange a été agité pendant 24 h à 22°, puis il a été neutralisé avec NaOH 2N, extrait et distillé pour fournir 3,7 g (16,8 mM) du produit désiré sous forme d'un mélange d'époxydes (rend. 84%).
IR : 1400 $cm^{-1}$ ;
Le mélange obtenu possédait une odeur boisée puissante.

L'époxydation a été également effectuée au moyen d'acide peracétique en opérant ainsi :

28 g (0,137 M) du mélange de cyclisation, obtenu sous lettre a. ci-dessus, ont été dilués dans 140 ml de $CH_2Cl_2$. Après adjonction de 24,8 g (0,3 M) d'acétate de sodium, on a additionné goutte à goutte en 2 h, à +5°, 22,4 ml (0,137 M) d'acide peracétique (47%) et le mélange a été ensuite agité 12 h à température ambiante. Après dilution avec $CH_2Cl_2$, neutralisation, extraction et distillation, on a obtenu 21,3 g (97 mM) du mélange désiré d'époxydes (rend. 71%).

## Exemple 2

### Parfumage de savon

100 G de pâte de savon en copeaux, obtenue à partir d'une base non parfumée de savon au sodium dérivé d'huile de noix de coco et de suif, ont été mélangés avec le produit obtenu à l'Exemple 1, additionné à raison de 0,1 g. Une fois homogénéisé, la pâte de savon obtenue a servi à préparer des savonnettes, lesquelles ont été soumises à une évaluation olfactive de la part d'un groupe d'experts. Ces derniers ont établi que les savonnettes parfumées possédaient une odeur bien boisée qui servait à couvrir parfaitement les odeurs peu agréables de la pâte à savon de base.

Exemple 3

Eau de toilette

On a préparé une composition parfumante de base de type Eau de Cologne masculine en mélangeant les ingrédients suivants (parties en poids) :

| | |
|---|---|
| Essence de sauge sclarée | 20 |
| Essence de lavande | 150 |
| Bergamote synthétique | 200 |
| Essence de citron | 140 |
| Essence d'orange douce | 40 |
| Galbanum synthétique à 10% * | 20 |
| Muscone à 10% * | 50 |
| 2-Pentyl-3-oxo-cyclopentylacétate de méthyle | 10 |
| 1,1-Diméthyl-6-tert-butyl-4-acétylindane | 10 |
| α-Isométhylionone | 50 |
| Ylang synthétique | 80 |
| Jasmin synthétique | 25 |
| Géranium synthétique | 50 |
| Néroli synthétique | 100 |
| Essence de coriandre | 5 |
| Phtalate de diéthyle | 50 |
| Total | 1000 |

* dans le phtalate de diéthyle

En ajoutant à 95 g de la composition de base ci-dessus 5 g du produit obtenu selon l'Exemple 1, on obtient un effet boisé net et caractéristique.

Exemple 4

On a préparé une composition parfumante de base en mélangeant les ingrédients suivants (parties en poids) :

| | |
|---|---|
| AMBROX® 1) 2) 10% * | 100 |
| Dimétol® 3) 10% * | 50 |

| | |
|---|---|
| Farenal 10% * | 50 |
| Ald. Hexylcinnamique | 150 |
| MAYOL® 1) 4) | 200 |
| Essence d'oliban | 10 |
| Parmanthème 1) 5) 10% * | 10 |
| Oxyde de rose 1) 10% * | 30 |
| Essence de thym rouge | 25 |
| Essence de jasmin synth. | 150 |
| Essence de rose synth. | 50 |
| Essence de lilas synth. | 25 |
| Total | 850 |

* dans le glycol dipropylique

1) origine : Firmenich SA, Genève

2) 3,6,6,9-tétraméthyl-perhydronaphto[2,1-b]furanne

3) origine : L. Givaudan & Cie, Vernier

4) 4-isopropyl-cyclohexyl-méthanol

5) composition à base de 2,6-nonadiénal

Si l'on ajoute à 85 g de la composition ci-dessus 15 g du produit obtenu selon l'Exemple 1, on obtient une nouvelle composition dont le caractère boisé-ambré se développe pleinement en rendant la composition plus harmonieuse.

0172376

## REVENDICATIONS

1. Composé oxygéné tricyclique de formule

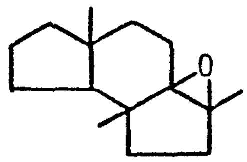

(I)

2. Utilisation du composé oxygéné tricyclique selon la revendication 1 à titre d'ingrédient parfumant.

3. Composition parfumante résultant de l'utilisation selon la revendication 2.

4. Procédé pour la préparation du composé selon la revendication 1, caractérisé en ce qu'on cyclise en milieu acide le 1,5,10-triméthyl-cyclododéca-1c,5t,9t-triène et époxyde ensuite le produit obtenu au moyen d'un peracide organique.

5. Procédé selon la revendication 4, caractérisé en ce que la cyclisation s'effectue à l'aide d'un mélange d'acide acétique et d'acide sulfurique et que l'époxydation s'effectue au moyen d'acide m-chloro-perbenzoïque.

Office européen des brevets

# RAPPORT DE RECHERCHE EUROPEENNE

0172376
Numero de la demande
EP 85 10 8418

## DOCUMENTS CONSIDERES COMME PERTINENTS

| Catégorie | Citation du document avec indication, en cas de besoin, des parties pertinentes | Revendication concernee | CLASSEMENT DE LA DEMANDE (Int Cl 4) |
|---|---|---|---|
| A | DE-B-2 461 594 (TAKASAGO PERFUMERY CO. LTD.) * revendications; colonne 2, lignes 23-30,53-58; colonne 3, ligne 17 - colonne 6, ligne 19 * --- | 1-5 | C 07 D 303/06<br>A 61 K 7/46<br>C 07 D 301/14<br>C 07 B 37/10<br>C 07 C 13/553 // |
| A | GB-A-1 152 932 (INTERNATIONAL FLAVORS AND FRAGRANCES INC.) * exemples I, IV, VII, VIII, XI * --- | 1-4 | |
| A | PATENT ABSTRACTS OF JAPAN, vol. 1, no. 48 (C-77), 11 mai 1977, page 6 C 77; & JP - A - 52 3837 (TAKASAGO KORYO KOGYO K.K.) 01-12-1977 --- | 1-4 | |
| A | TETRAHEDRON, vol. 35, no. 11, 1977, pages 1463-1467, Londres, GB; F. TURECEK et al.: "Structures of the tricyclododecenes prepared by the catalytical intramolecular cyclisation of 1,5,9-cyclododecatriene" --- -/- | 1,4 | **DOMAINES TECHNIQUES RECHERCHES (Int Cl 4)**<br>A 61 K 7/46<br>C 07 C 5/31<br>C 07 C 13/00<br>C 07 D 301/00<br>C 07 D 303/00 |

Le présent rapport de recherche a été établi pour toutes les revendications

| Lieu de la recherche | Date d'achèvement de la recherche | Examinateur |
|---|---|---|
| BERLIN | 24-10-1985 | HASS C V F |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503 03 82

Office européen des brevets

# RAPPORT DE RECHERCHE EUROPEENNE

0172376

Numéro de la demande

EP 85 10 8418

Page 2

| DOCUMENTS CONSIDERES COMME PERTINENTS | | | |
|---|---|---|---|
| Catégorie | Citation du document avec indication, en cas de besoin, des parties pertinentes | Revendication concernee | CLASSEMENT DE LA DEMANDE (Int Cl 4) |
| A | CHEMICAL ABSTRACTS, vol. 75, no. 7, 16 aout 1971, page 318, abrégé no. 48535d, Columbus, Ohio, US; M. TICHY et al.: "Stereochemical studies. LVIII. Synthesis of trans-anti-trans-perhydro-as-indacene, a system containing a "frozen" cyclohexane chair", & COLL. CZECH. CHEM. COMMUN., vol. 36, no. 4, 1971, pages 1426-1435 --- | 1,4 | |
| A | PATENT ABSTRACTS OF JAPAN, vol. 4, no. 166 (C-31)[648], 18 novembre 1980; & JP - A - 55 108 864 (KAO SEKKEN K.K.) 21-08-1980 --- | 2-4 | |
| A | DE-B-1 693 162 (FIRMENICH S.A.) * colonne 10, ligne 50 - colonne 12, ligne 68 * ----- | 3,4 | DOMAINES TECHNIQUES RECHERCHES (Int Cl 4) |

Le présent rapport de recherche a été établi pour toutes les revendications

| Lieu de la recherche | Date d achèvement de la recherche | Examinateur |
|---|---|---|
| BERLIN | 24-10-1985 | HASS C V F |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire
T : théorie ou principe à la base de l'invention
E : document de brevet antérieur, mais publié à la date de dépôt ou après cette date
D : cité dans la demande
L : cité pour d'autres raisons
& : membre de la même famille, document correspondant

OEB Form 1503 03 82